Europäisches Patentamt

European Patent Office

Office européen des brevets

(19)

(11) Publication number:

0 388 782
A1

# EUROPEAN PATENT APPLICATION

(12)

(21) Application number: 90104866.0

(22) Date of filing: 15.03.90

(51) Int. Cl.5: G01N 33/52

(30) Priority: 20.03.89 US 326094

(43) Date of publication of application:
26.09.90 Bulletin 90/39

(84) Designated Contracting States:
DE FR GB

(71) Applicant: QUANTAI BIOTRONICS INC.
15405 Redhill Avenue, Suite A & B
Tustin, CA 92680(US)

(72) Inventor: Shuenn-Tzong Chen
Two Tory
Irvine California 92714(US)

(74) Representative: Schmidt-Bogatzky, Jürgen, Dr.
Ing. et al
Schlossmühlendamm 1
D-2100 Hamburg 90(DE)

(54) Method for determination of analytes.

(57) A multilayer analysis film (13) for analyzing liquid samples is described. The multilayer analysis film (13) is arranged in a sandwich format between two plastic supports (6, 8). The upper plastic support (6) is laminated to the surface of the multilayer analysis film (13) with an open air space (5) in between providing the necessary capillary action for the liquid sample to spread uniformely over the multilayer analysis film (13). This capillary action has ability to supply liquid sample on the surface thereof to the reagent containing layer at a constant volume per unit area without employing a spreading layer. The method is particularly suitable for the analysis of an analyte in whole blood sample.

Fig.1

EP 0 388 782 A1

## BACKGROUND OF THE INVENTION

The present invention relates to a multilayer analysis film for analyzing liquid samples. The use of the spreading layer in a multilayer analysis film was first described in the U.S. Patent No. 3,492,158. Such a device is said to be useful in analyzing liquid sample components, such as glucose, cholesterol, and others. The 158 patent provided for a multilayer analysis film comprising a spreading layer which is made from non-fibrous material, a radiation blocking layer, a reagent layer, and a light transmitting layer. Upon liquid sample application to the surface of the multilayer analysis film, the spreading layer, at its outer position, has the ability to spread and supply the sample to the reagent containing layer at an approximately constant volume per unit area. This spreading property is very useful and allows for a liquid sample to be analyzed quantitatively without precise measurement of the volume

However the 158 patent has certain drawbacks. For example, in manufacturing the porous spreading layer, it is technically difficult to control the particle size, volume ratio and so on using non-fibrous material, such as cellulose acetate, to produce a very uniform film.

This problem is further addressed by the patentees of U.S. Patent No. 4,292,272. This discloure deals with a fabric material which is laminated. to a multilayer analysis film in place of the non-fibrous porous spreading layer as mentioned in the 158 patent. This fabric is said to also have an ability to spread the liquid sample uniformly to the reagent containing layer the same way as the patent 158's spreading layer.

However, the 272 patent also has a significant drawback. In preparing the multilayer analysis film using fabric as the spreading layer, it is often technically difficult to place the fabric onto the multilayer analysis film uniformly. The bonding between the fabric and multilayer analysis film is usually accomplished by employing an adhesive layer. For example, a 1% gelatin solution is applied as the adhesive layer. This adhesive layer tends to hinder the diffusion of the larger molecules present in the liquid sample. Furthermore, in order to assure tight bonding between the fabric layer and multilayer analysis film, some pressure is applied when pressing these two together. This pressure sometimes causes some damage to the multilayer analysis film.

Accordingly, there remains a need for a system of a multilayer analysis film for analyzing liquid sample, that employs a spreading layer which is easier to handle and manufacture.

## SUMMARY OF THE INVENTION

Therefore in view of the foregoing, it is an objective of subject invention to provide a simple means for spreading liquid sample using multilayer analysis film without incurring the problems mentioned above.

Thus according to the subject invention, the multilayer analysis film for analyzing the liquid sample comprises, in sequence, a lower plastic support, an adhesive layer, a water-impermeable light transmitting layer, a reagent layer, a non-porous reflectance layer, and the upper plastic support. The upper plastic support is laminated to the surface of the multilayer analysis film with an open air space in between providing the necessary capillary action to spread the liquid sample. There is no liquid spreading layer utilized in this invention.

According to the invention it is devised a simple, novel method for spreading the liquid sample efficiently by employing two plastic supports to sandwich the multilayer analysis film. This fact must be regarded as being the more surprising since, the above patents purposely construct a spreading layer as an essential element for multilayer analysis film.

## BRIEF DESCRIPTION OF THE DRAWINGS OF THE INVENTION

Figure 1 is a schematic cross-section diagram of the invention. The numbers therein indicate the following components of the invention. 1. a reflectance layer, 2. a reagent layer, 3. a light transmitting layer, 4. the location on which a liquid sample is applied, 5. the air space that seperates the reflectance layer and the upper plastic support, 1 and 6, and the location at which a liquid sample spreads and absorbs uniformly. 6. the upper plastic support, 7. the adhesive layer, 8. the lower plastic support, and 9. the direction from which observations are made.

Figure 2 is the overall configuration of the invention. The multilayer analysis film is sandwiched between two plastics supports. The numbers therein correspond to the components described above.

Figure 3a to 3c depict the cross-section diagram of the other preferable multilayer analysis film compositions, each of which is produced in accordance with an embodiment of this invention. The number 10 therein indicates a second reagent layer and number 11 indicates a filter layer.

## DETAILED DESCRIPTION OF THE INVENTION

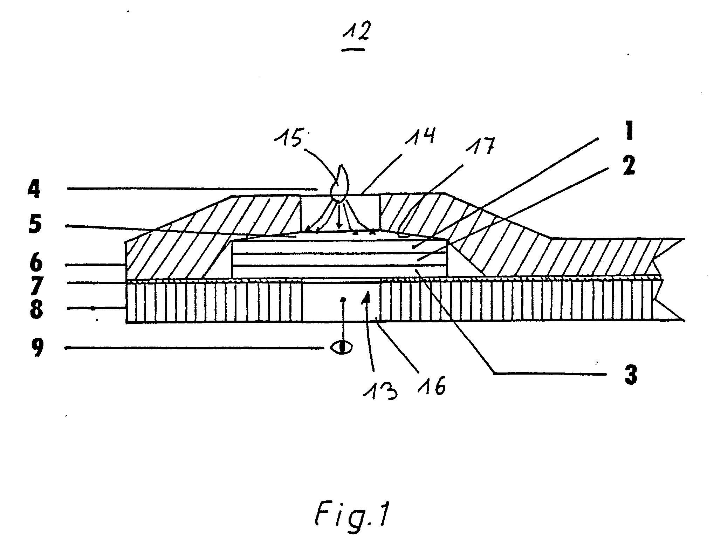

The present test device 12 comprises four basic elements as shown in Figure 1 : a lower plastic support 8, an adhesive layer 7, multilyer analysis film 13, and an upper plastic support 6. The multilayer analysis film 13 consists of a light transmitting layer 3, at least one reagent layer 2, and a reflectance layer 1. The light transmitting layer is affixed to the lower plastic support through the adhesive layer. The reagent layer 2 containing the reagent system responsive to the components to be analyzed is coated on the light transmitting layer 3. And finally, the reflectance layer 1 is coated on the reagent layer 2.

As shown in Figure 1, wherein the cross section diagramm of this invention is illustrated, the upper plastic support 6 is affixed to the surface of the multilayer analysis film 13 by the adhesive layer 7 at both ends of the plastic supports, and not directly on the multilayer analysis film 13. There is an open air space 5 between the multilayer analysis film 13 and the upper plastic support 6, i.e., there is no bonding between the upper plastic support 6 and the surface of the multilayer analysis film 13. The upper and lower plastic support layers are affixed to each other by the adhesive layer 7 at both ends of the two plastic supports. The end with the multilayer analysis film 13 has a hole 14 at 4mm or 5mm in diameter at the sample application site 4. The other end serves as a convenient handle. The test device 12 can be held at the handle end, while the other end containing the multilayer analysis film 13 is contacted with the liquid sample 15 through the sample application hole 14. The opposite side of the lower plastic support serves as the color observation site 9. Materials that may be used for the support members include a myriad of plastic or polymer. Examples include such polymeric materials as cellulose acetate, polyester, polycarbonate, polyvinylchloride, polyethylene and polystyrene. The support can be opaque or it can transmit light. The plastic support can also be hydrophilic or hydrophobic in nature.

In preparation of this invention, it is necessary to have the open air space in between the upper plastic support 6 and the multilayer analysis film 13 to measure 0.1 to 1000 microns, preferably between 1 to 25 microns. It is found that an air space 5 measuring in this range provides the best ability to spread the liquid sample uniformly onto the multilayer analysis film 13. If there is no air space in between, then the spreading ability ceases. It is also important to have the size of the hole 14 where the sample is applied to be between 1 to 10 mm in diameter, preferably between 2 to 5 mm to provide the optimum capillary action to spread liquid sample. This hole size is found to be optimal in absorbing 10 to 20 microliters of the liquid sample.

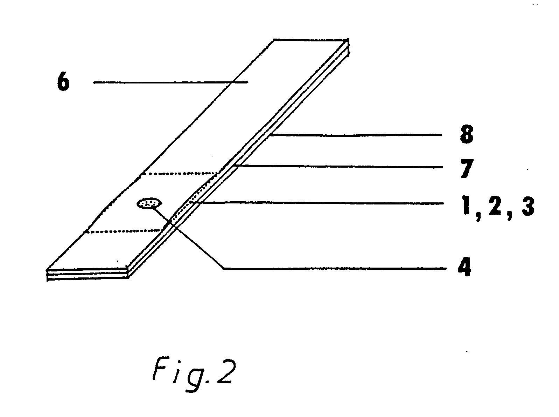

The adhesive layer 7 can be composed of any adhesive capable of adhering the plastic support and the multilayer analysis film 13. This adhesive layer 7 is coated on the lower and not on the upper plastic support. Examples of adhesive that can be used include gelatin, rubber, silicone and acrylate base glue. In preparation of this invention, it is a common practice to first laminate the two plastic supports together with the adhesive. The plastic supports are cut into strips measuring of 0.5 inch in width and 2 inch in length. A 4 to 5 mm diameter hole is punched near the end of the plastic supports. The upper plastic support 6 is then partially peeled off from the end containing the hole 14. The mulitlayer analysis film 13 cut into a rectangular dimension of 0.5 inch by 0.625 inch is placed over the hole on the lower plastic support 8 and is adhered thereto by the adhesive. The upper plastic support 7 is then placed back over the surface of the multilayer analysis film 13. A slight pressure is applied to the surface of the plastic supports to ensure strong bonding between the two plastic supports and the multilayer analysis film 13. It is necessary to exactly align the holes 14, 16 on both plastic supports. Thus, a test device 1 of the invention is constructed. A diagram illustrating this configuration is shown in Figure 2.

In use, a drop of the liquid sample 15 is applied to the sample application site 4 as shown in Figure 1. The capillary action occurs at gap 17 between the upper plastic support 6 and the multilayer analysis film 13. This capillary action provides the force to uniformly spread the liquid sample onto the multilayer analysis film 13 as shown by the direction of the arrows and allows the sample components to pass through the refectance layer 1 to reach the reagent layer 2 by nearly the same fraction of volume per unit area. Consequently, a certain reaction occurs in the reagent layer 2 resulting in an uniform color formation or color change. After a finite time, this color response is observed from to opposite side of the lower plastic support 8. Therefore, the concentration of a specific component in a liquid sample 15 can be determined colormetrically. The air space 5 not only provides the ability to spread the sample 15 in a constant volume per area but it also can hold in the excess void volume up to 10 to 90% of the total sample volume. In this way, it is found that the volume size of the liquid sample 15 is not critical for reaction by the multilayer analysis film 13.

It should be noted that the multilayer analysis film 13 used here is very similar to those employed in U.S. patents 3,992158 and 4,292,272 except for the fact that a spreading layer is not used. Therefore, in principle, any multilayer analysis film 13 can be utilized here. Thus, a typical multilayer analysis film 13 here will contain a light transmitting layer 3, and at leat one reagent layer 2.

The materials and construction of the respective individual layers of the multilayer analysis film 13 used in this invention are described in greater detail below.

A light transmitting layer 3 is a plastic film support capable of transmitting electromagnetic radiation of a wave-length of about 200 nm to 900 nm. Also this transmitting layer is not water-impermeable in nature.

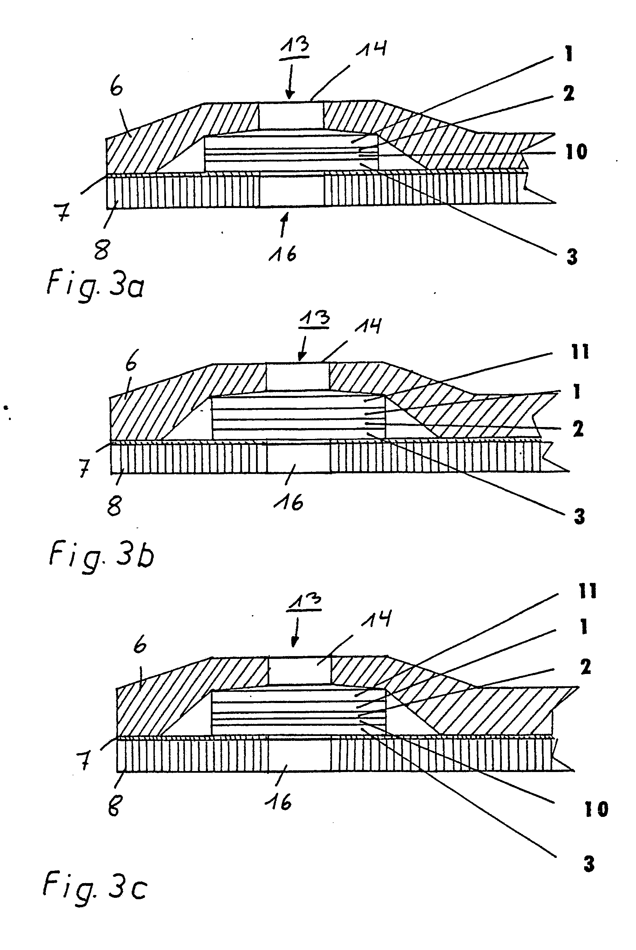

The reagent layer 2 is contained in a layer made of a binder agent such as gelatin or any other hydrophilic polymers. The reagents employed depends on the ultimate analysis to be performed with the test device 12. If the substance to be analyzed is glucose, the reagents are any one of many known formulations which produce a colorimetric or other detectable response to the glucose. A preferred reagent for analyzing glucose comprises glucose oxidase, peroxidase and a redox indicator. Redox indicators suitable for such a glucose sensitive reagent include o-tolidine, o-dianisidine, $3,3',5,5'$-tetramethylbenzidine, 4-aminoantipyrine and others known in the art. They are capable of being oxidized, in the presence of hydrogen peroxide and peroxidatively active substance, to produce a colored product. Other reagents usable in the invention include those capable of detecting uric acid, cholesterol and others. For cholesterol, a suitable reagent system could comprise cholesterol esterase, cholesterol oxidase, peroxidase and a redox indicator. In a preferred reagent format for glucose determination, the glucose oxidase, peroxidase and o-tolidine can be incorporated into the gelatin mix and coated onto the light transmitting layer 3. If necessary, two or three coating solutions can be employed, such that the reagent system gives the maximum perfomance. Such a multiple coating process enables the isolation of reagent system ingredients which are incompatible with one another in solution, but which are compatible in a dry state. This is shown in Figure 3 a), wherein a second layer 10 is indicated.

Following the reagent layers, a non-porous reflectance layer 1 may be applied to the system. In this invention, this layer may or may not be included. The multilayer analysis film 13 may comprise only of the light transmitting layer 3 and the reagent layer 2. This two layer analysis film can then be laminated with the two plastic supports as previously shown. The configuration is found to spread the liquid sample in the same manner as three layers containing the reflectance layer 1. However, for practical purposes, it is desirable to include a reflectance layer 1 to shield the interference from the samples. The reflectance layer 1 can be prepared by coating a dispersion of fine titanium oxide powder or any other white pigments in a water-permeable hydrophilic polymer binder in a layer having a thickness of from 5 to 100 micrometers which permits the passage of liquid components therethrough. Examples of some white pigments include titanium dioxide, zinc oxide, barium sulfate and aluminium, etc.

It is also useful to include a filter layer 11 in the multilayer analysis film 13. Figure 3 b) shows this diagram. A filter layer 11 is laminated on the surface containing the light transmitting layer 3, the reagent layer 2, and the reflectance layer 1. The filter layer 11 here serves to separate the formed components of the liquid sample such as whole blood sample, and allow the passage of only the fluid part to the underlying layers. The filter layer 11 may be made of, for example, sheet like filter material composed of powders or fibers such as synthetic or natural fibers and glass fibers, or any filter membranes having suitable pore size. The filter layer 11 seperates the formed components of the blood at one time, or successively, for example in the order of leucocytes, erythrocytes, and platelets. The pore size of the filter layer 11 should be preferably between 0.5 to 10 microns, with the best result achieved between 1.2 and 5 microns. The thickness of the filter layer 11 is from approximately 5 microns to about 1 mm, with the best result achieved between 100 microns to about 1 mm. To prevent hemolysis of the whole blood sampel on .contact with the filter layer 11, the material comprising the filter layer 11 may be treated with chemicals, such as those disclosed in U.S. Patent No. 3,552,928. For example, water soluble amino acids are effective in causing seperation of the colored red cells from the whole blood. Specific examples of the filter layer 11 are a layer composed of a laminate of membrane filter having a pore size of 1.2 micron in diameter with a thickness of 100 microns. Materials suitable for such filter layer 11 include LoProdyne manufactured form Pall Corporation, glass fiber £257 from Schleicher and Schuell.

Furthermore, the filter layer 1 can also be treated with chemicals such as inorganic salt to enhance the binding of certain components in liquid samples to the filter membrane layer. Examples of these inorganic salts include quarternary ammonium and carboxy groups to provide a positive or negative charge on the filter layer 11.

In addition, the filter layer 11 can also be treated with some surfactants such as Triton X100 (Rohm and Haas) with concentration from 0.1% to 2.0% to provide the surface wetting function.

Furthermore, the filter layer 11 can also be laminated to the multilayer analysis film 13 containing two or more reagent layers as shown in Figure 3 c), wherein the number 10 indicates the second reagent layer and 11 indicates the filter layer.

## EXAMPLE 1

Each of three layer analysis film was prepared as follows: a reagent layer containing 500 units per square meter of glucose oxidase, 2500 units of peroxidase per square meter. 1.0 gram of 0-tolidine dihydrochloride per square meter, and 7.5 grams of gelatin per square meter was coated on a 4 mil polyester transparent plastic support and dried. A non-porous reflectance layer consisting of 35 grams of titanium dioxide per square meter and 7.5 grams square meter of gelatin was coated on the above layer and dried. A 7 mil white polyester plastic film was first coated with 1% acrylate adhesive and was laminated to a 2 mil polyethylene film. These plastic films were cut into half inch wide and 2 inch long strips as handles. On one end of this plastic handle, a 4 mm diameter hole was punched. Then, the upper 2 mil polyethylene film was partially peeled from the end with the hole and the above three layer analysis film was placed over the hole and adhered onto the 7 mil polyester handle. The 2 mil polyethylene film was then placed back over the three layer analysis film and adhered to the 7 mil white polyester plastic film by the adhesive at the other end and pressed through a press roll to assure proper adhesion. The final configuration can be compared in Figure 1 and 2. Thus an integral three layer analysis film was prepared with an open air space between the 2 mil polyethylene plastic film and the surface of the three layer analysis film. The air space is measured at about 25 microns in width. A serum sample size of 10 microliters was applied to the hole of this three layer analysis film. This analysis film was compared to a regular three layer analysis film without the two plastic handles for spreading properties. In twenty seconds, the diameter and the area were measured and the results were as following:

|  | Film Only | Film Using This Invention |
|---|---|---|
| Spot Diameter (in cm) | 0.6 | 1.3 |
| Area (in square cm) | 0.282 | 1.33 |

The analysis film using this invention is useful for the detection of glucose in serum with the presence of glucose being indicated by the formation of the color blue in the reagent layer.

## EXAMPLE 2

The same reagent layer and reflectance layer were prepared as in Example 1. To form a multilayer analysis film for the subject invention to test whole blood samples, a filter layer was laminated to the above film. This filter membrane can be any commercial hydrophilic nylon membrane, cellulose acetate membrane or nitrocellulose membrane possessing no spreading properties. An example is a membrane filter sold by Pall Corporation under the name of LoProdyne having an average pore size of 1.2 micron. The lamination was carried out by first immersing the membrane in the citrate buffer (pH = 5.0) for twenty minutes, then placing this membrane over the above multilayer analysis film and applying slight pressure to increase the adhesion. This filter layer served to seperate for the red blood cells from the whole blood sample and has no spreading purpose. This multilayer analysis film was then inserted into two plastic supports in the same manner as in Example 1. A 10 microliter whole blood sample was deposited onto the hole of this invention to test the spreading properties. An identical multilayer analysis film without the two plastic supports was used as a comparison. In less than twenty seconds, the spreading ability was assessed by measuring the diameter and area of the spot. The results were as follows:

|  | Film Only | Film Using This Invention |
|---|---|---|
| Spot Diameter (in cm) | 0.9 | 1.4 |
| Area (in square cm) | 0.78 | 1.53 |

The analytical film using this invention is useful for the detection of glucose in whole blood samples. The formation of the color blue indicates the presence of glucose while the red blood cells are screened by the filter layer. This multilayer analysis film prepared as above was tested with whole blood samples containing glucose concentration ranging from 80 to 415 mg/dl. The color formation is quantified with a Macbeth 1500 Reflectance Spectrophotometer manufactured by Macbeth Company. The wavelength at 660 nm was used after one minute incubation at room temperature. The results were as follows:

| Whole Blood Glucose (mg/dl) | Optical Density |
|---|---|
| 80 | 0.32 |
| 179 | 0.54 |
| 254 | 0.62 |
| 319 | 0.72 |
| 415 | 0.84 |

It is, therefore, concluded that the subject invention is useful for determination of the analytes in whole blood sample.

While the invention has been described in detail and with reference to specific embodiments thereof, it will be apparent to one skilled in the art that various changes and modifications can be made therein without departing from the spirit or scope thereof.

## Claims

1. Multilayer analysis film (13) for analyzing liquid samples, characterized in that in sequence, a non-spreading refectance layer (1), at least one ragent layer (2) and a water-impermeable light transmitting layer (3), being laminated in a sandwich format to two plastic supports (6, 8) or any other suitable materials, having an air space (5) between the surface of the multilayer analysis film (13) and the upper plastic support (6), providing capillary action with an ability to spread the liquid sample (15) to the reagent containing layer at a constant volume per unit area without employing a spreading layer.

2. Multilayer analysis film (13) according to claim 1, characterized in that capillary action has the characteristics of spreading the serum sample onto the surface of the multilayer analysis film (13) in less then twenty seconds.

3. Multilayer analysis film (13) according to claim 1, characterized in that air space between the upper plastic support (6) and the multilayer analysis film (13) is from 0.1 micron to 10 mm, preferably from 1 to 50 microns.

4. Multilayer analysis film (13) according to claim 1, characterized in that the two plastic supports (6, 8) are polyvinylchloride, polyester, polyethylene or any material capable of laminating the multilayer analysis film (13) with an air space (5) between the top plastic support (6) and the multilayer analysis film (13).

5. Multilayer analysis film (13) according to claim 1, characterized in that the non-porous reflectance layer (1) contains pigment such as titanium dioxide, zinc oxide or barium sulfate.

6. Multilayer analysis film (13) according to claim 1, characterized in that there contains at least one reagent layer (2) present in a water-permeable hydrophilic binder such as gelatin.

7. Multilayer analysis film (13) according to claim 1, characterized in that the analytical film support is a light transmitting layer (3) which is capable of transmitting the light.

8. Multilayer analysis film (13) according to claim 1, characterized in that the adhesive layer (7) is acrylate, rubber, silicone, gelatin or any other adhesive capable of adhering to the surface of the plastic support (8).

9. Multilayer analysis film (13) for analyzing liquid samples characterized in that in sequence, a wettable, non-spreading filter layer (11), a non-porous reflectance layer (1), at least one reagent layer (2) and a water-impermeable light transmitting layer (3) being laminated in a sandwich format to plastic supports (6, 8) or any other suitable materials, having an air space (5) between the surface of the membrane filter layer (11) and the upper plastic support (6) providing a capillary action with a ability to spread the liquid sample to the reagent containing layer at a constant volume per unit area without employing a spreading layer.

10. Multilayer analysis film (13) according to claim 9, characterized in that capillary action has the characteristics of spreading the whole blood samples in less than twenty seconds.

11. Multilayer analysis film (13) according to claim 9, characterized in that the filter layer (11) is glass fiber, or any other wettable, non-spreading filter membrane with pore size ranging form 0.45 to 12 microns, preferably between 1.2 to 5 microns.

12. Multilayer analysis film (13) according to claim 9, characterized in that the filter layer (11) is treated with 0.1% to 2% surface acting agent.

13. Multilayer analysis film (13) according to claim 9, characterized in that the filter layer (11) is treated with 0.1% to 5% amino acid solution.

14. Multilayer analysis film (13) according to claim 9, characterized in that the filter layer (11) is capable of seperating the red blood cells and allowing the serum to diffuse through the multilayer analysis film (13) to contact with the reagent layer (2).

15. Multilayer analysis film (13) according to claim 9, characterized in that the reagent layer (2) is capable of interacting with the liquid sample analyte to produce the color response.

16. Multilayer analysis film (13) according to claim 9, characterized in that the color response may be observed with the reflectance photometer having a wavelength from 200 to 900 nm.

<u>12</u>

Fig.1

**6**

**8**

**7**

**1, 2, 3**

**4**

*Fig.2*

Fig. 3a

Fig. 3b

Fig. 3c

European Patent
Office

**EUROPEAN SEARCH REPORT**

Application Number

EP 90 10 4866

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 212 634 (MILES LABORATORIES) <br> * Column 2, lines 11-14; column 3, lines 24-43 * | 1,4,6,7 ,9-11, 15,16 | G 01 N 33/52 |
| Y | EP-A-0 010 456 (EASTMAN KODAK CO.) <br> * Page 2, lines 28-36; page 8, line 20 - page 10, line 11; figure 2 * | 1-16 | |
| Y | EP-A-0 231 127 (EASTMAN KODAK CO.) <br> * Page 34, example 6 * | 1-16 | |

TECHNICAL FIELDS
SEARCHED (Int. Cl.5)

G 01 N

The present search report has been drawn up for all claims

| Place of search | Date of completion of the search | Examiner |
|---|---|---|
| THE HAGUE | 13-06-1990 | VAN BOHEMEN C.G. |